# EUROPÄISCHE PATENTSCHRIFT

(11) **EP 0 821 960 B1**
(45) Veröffentlichungstag und Bekanntmachung des Hinweises auf die Patenterteilung: **09.04.2003**
(21) Anmeldenummer: 97112939.0
(22) Anmeldetag: 28.07.1997
(51) Int. Cl.: A61K 31/52, A61K 31/42, A61K 31/275, A61P 9/00, A61P 11/00, A61P 13/00, A61P 17/00, A61P 21/00, A61P 25/00, A61P 29/00, A61P 33/00, A61P 35/00, A61P 37/00

(54) **Verwendung von Xanthinderivaten in Kombination mit einem 5-Isoxazol-4-carbonsäureamid Derivat und/oder einem 2-Cyano-3-hydroxy-crotonsäureamid Derivat zur Modulation der Apoptose**
Use of xanthine derivatives combined with a 5-substituted-isoxazole-4-carboxamide derivative and/or a 2-cyano-3-hydroxycrotonamide derivative for the modulation of apoptosis
Utilisation de dérivés de xanthine associés à un dérivé d'isoxazole-4-carboxamide-5-substitué et/ou un dérivé 2-cyano-3-hydroxycrotonamide pour la modulation de l'apoptose

(30) Priorität: 31.07.1996 DE 19630837; 01.10.1996 DE 19640556
(43) Veröffentlichungstag der Anmeldung: 04.02.1998
(73) Patentinhaber: HOECHST AKTIENGESELLSCHAFT, 65929 Frankfurt am Main (DE)
(72) Erfinder: Müllner, Stefan, Dr., 65239 Hochheim (DE); Dax, Claudia, DCh., 64579 Gernsheim (DE)

(56) Entgegenhaltungen:
- EP-A- 0 279 079
- EP-A- 0 514 789
- EP-A- 0 528 164
- EP-A- 0 547 508
- EP-A- 0 557 876
- EP-A- 0 665 013
- WO-A-92/07566
- WO-A-95/10282
- WO-A-96/20710
- US-A- 4 833 146
- DATABASE WPI Week 9630 Derwent Publications Ltd., London, GB; AN 96-151131 XP002050915 & WO 96 05836 A (UNIV. SOUTHERN CAROLINA) , 29.Februar 1996
- DATABASE WPI Week 9340 Derwent Publications Ltd., London, GB; AN 93-320439 XP002050916 & WO 93 18770 A (UNIV. SOUTHERN CALIFORNIA) , 30.September 1993
- DATABASE WPI Week 9252 Derwent Publications Ltd., London, GB; AN 92-433353 XP002050917 & WO 92 21344 A (HUTCHINSON CANCER RES. CENT. FRED.) , 10.Dezember 1992
- DATABASE WPI Week 9004 Derwent Publications Ltd., London, GB; AN 90-024285 XP002050918 & EP 0 351 885 A (HOECHST AG) 24.Januar 1990

## Beschreibung

Bei der Apoptose handelt es sich im Gegensatz zur Nekrose um einen genetisch kontrolliert verlaufenden (programmierten) Zelltod, der essentieller Bestandteil des Lebens mehrzelliger Organismen ist.

Im Gegensatz zu diesem normalen und lebensnotwendigen Apoptoseprozess sind zahlreiche Krankheitsformen oder deren Symptome Ausdruck einer abnormalen, d.h. a) ausufernden oder b) unterdrückten Apoptose [a): Infarkt, Stroke oder Neurodegeneration, b) hypertrophische Erkrankungen]. Heilungsvorgänge von Krankheiten können somit durch Unterdrückung oder Aktivierung der Apoptose möglich sein (z. B. Querschnittslähmung, Immunabwehr usw.). Apoptose verläuft nach Induktion definierter Todessignale, beispielsweise durch Stimulation bestimmter Rezeptoren (z. B. Fas-Rezeptor), über eine sekundär induzierte komplexe Kaskade von ineinandergreifenden biochemischen Ereignissen, an deren Ende die Auflösung der intakten Zelle zu Membran-abgepackten Einheiten steht, die vom Körper ohne oder nur mit geringem Schaden für die umliegenden Zellen (Gegensatz zur Nekrose) entsorgt werden können. Dabei sind in manchen Fällen die Übergänge zwischen Nekrose und Apoptose fließend; so gibt es Fälle, in denen Nekrose zur Apoptose (oder umgekehrt) führt (z. B. Infarkt, Stroke etc.).

Cofilin, ein 19 kDa großes aktinbindendes Protein, spielt als costimulatorischer Faktor in T-Zellen eine entscheidende Rolle bei der Immunreaktion. Cofilin liegt im Cytosol phosphoryliert vor und wird nach Dephosphorylierung in den Zellkern transportiert. Hierbei dient es offenbar als Schleppermolekül für das Protein Aktin, welches keine nukleare Erkennungssequenz besitzt und als DNAse-I-Inhibitor bekannt ist. Durch diesen Mechanismus kann der Phosphorylierungsgrad des cytosolischen Cofilins einen regulierenden und modulierenden Einfluß auf die Apoptose von Zellen nehmen.

In der Internationalen Anmeldung WO 96 20 710 A werden Xanthinderivate und ihre Verwendung zur Herstellung von Arzneimitteln zur Hemmung der Zellapoptose beschrieben. Die Europäische Patentanmeldung EP-A-0 665 013 beschreibt N-Phenyl-isoxazol-carboxamid Derivate und N-Phenyl-cyanocrotonamid Derivate und ihre Verwendung zur Herstellung eines Arzneimittels zur Behandlung von Gefäßerkrankungen im Zusammenhang mit Autoimmunerkrankungen, Schlaganfall und Transplantationen.

Ein Kombinationspräparat, enthaltend ein Xanthinderivat der Formel I und eine Verbindung der Formel IV oder V zeigt einen überadditiven Hemmeffekt auf die Dephosphorylierung von Cofilin und damit auf die Aktivierung von Cofilin, die zu einer Modulation der Apoptose führt. Aufgrund des Ausmaßes dieses Effekts läßt sich die Anwendung dieses Kombinationspräparats auf Bereiche ausdehnen, die beispielsweise eine immunsuppressiven Therapie durch die Einzelkomponenten bislang verschlossen waren.

Die Erfindung betrifft daher ein Kombinationspräparat, enthaltend

### 1) mindestens ein Xanthinderivat der Formel I,

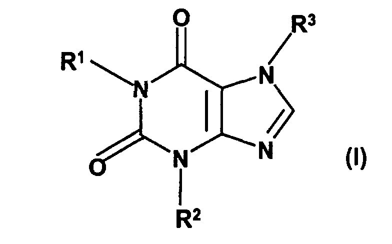

und/oder eine gegebenenfalls stereoisomere Form der Verbindung der Formel I,
wobei R² für -(C₁-C₄)-Alkyl steht,
einer der Reste R¹ oder R³ für einen Rest der Formel II steht,

-(CH₂)ₙ-R-CH₃ (II)

worin R für
   a) eine kovalente Einfachbindung steht und worin n die ganze Zahl Null, 1, 2, 3, 4, 5, 6 oder 7 bedeutet,
   b) einen Rest -CO- steht und worin n die ganze Zahl 1, 2, 3, 4, 5 oder 6 bedeutet, oder
   c) einen Rest -C(R⁴)(OH)- steht und worin n die ganze Zahl 1, 2, 3, 4, 5 oder 6 bedeutet und
R⁴ für
   a) einen Wasserstoffatom oder b) -(C₁-C₃)-Alkyl steht, und
der andere Rest R³ oder R¹ für
   a) ein Wasserstoffatom,
   b) (C₁-C₇)-Alkyl,
   c) (C₄-C₈)-Cycloalkyl-alkyl oder
   d) Alkyl mit 2 bis 6 Kohlenstoffatomen steht, worin die Kohlenstoffkette mit einem Sauerstoffatom unterbrochen ist,

### 2) eine Verbindung der Formel IV und/oder V,

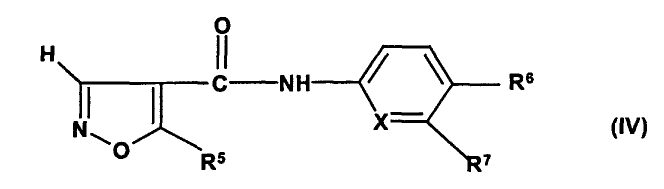

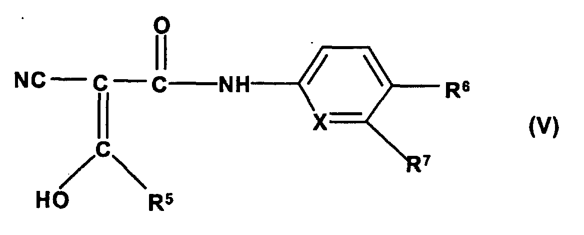

und/oder eine gegebenenfalls stereoisomere Form der Verbindung der Formel IV oder V und/oder ein physiologisch verträgliches Salz der Verbindung der Formel V, wobei
R⁵ für
   a) (C₁-C₄)-Alkyl,
   b) (C₃-C₅)-Cycloalkyl,
   c) (C₂-C₆)-Alkenyl oder
   d) (C₂-C₆)-Alkinyl, steht,
R⁶ für
   a) -CF₃,
   b) -O-CF₃,
   c) -S-CF₃,
   d) -OH,
   e) -NO₂,
   f) Halogen,
   g) Benzyl,
   h) Phenyl,
   i) -O-Phenyl,
   k) -CN oder
   l) -O-Phenyl, ein oder mehrfach substituiert mit
      1) (C₁-C₄)-Alkyl,
      2) Halogen,
      3) -O-CF₃ oder
      4) -O-CH₃, steht,
R⁷ für
   a) (C₁-C₄)-Alkyl,
   b) Halogen, oder
   c) ein Wasserstoffatom steht, und
X für
   a) eine -CH-Gruppe oder
   b) ein Stickstoffatom, steht, und

### 3) einen pharmazeutischen Träger.

Bevorzugt werden Verbindungen der Formel I eingesetzt, wobei
R² für (C₁-C₄)-Alkyl steht und
einer der Reste R¹ oder R³ für einen Rest der Formel II steht, worin R für
a) einen Rest -CO- oder
b) einen Rest -C(R⁴)(OH)- steht,
und n die ganze Zahl 3, 4, 5 oder 6 bedeutet und
R⁴ für ein Wasserstoffatom oder (C₁-C₃)-Alkyl steht und
der andere Rest R³ oder R¹ für (C₁-C₇)-Alkyl oder (C₄-C₈)-Cycloalkyl-alkyl steht.

Besonders bevorzugt werden Verbindungen der Formel I eingesetzt, wobei
R² für (C₁-C₂)-Alkyl steht,
R¹ für den Rest der Formel II steht, worin R für
a) einen Rest -CO- oder
b) einen Rest -C(R⁴)(OH)- steht,
und n die ganze Zahl 3, 4, 5 oder 6 bedeutet und
R⁴ für ein Wasserstoffatom oder (C₁-C₂)-Alkyl steht und
R³ für (C₁-C₇)-Alkyl oder (C₄-C₈)-Cycloalkyl-alkyl steht.

Insbesondere bevorzugt werden Verbindungen der Formel I eingesetzt, wobei
R² für (C₁-C₂)-Alkyl steht,
R¹ für einen Rest der Formel II steht, worin R für
a) einen Rest -CO- oder
b) einen Rest -C(R⁴)(OH)- steht,
und n die ganze Zahl 3, 4, 5 oder 6 bedeutet und
R⁴ für ein Wasserstoffatom oder (C₁-C₂)-Alkyl steht und
R³ für (C₂-C₅)-Alkyl oder (C₄-C₆)-Cycloalkyl-alkyl steht.

Ganz besonders bevorzugt wird 1-(5-Hydroxy-5-methylhexyl)-3-methyl-7-propylxanthin verwendet.

Die Alkylreste der Formel I sind geradkettig oder verzweigt. Der Ausdruck "(C₄-C₈)-Cycloalkyl-alkyl" definiert solche Alkylreste, die mit (C₃-C₆)-Cycloalkyl substituiert sind, wobei die Summe aller C-Atome kleiner oder gleich 8 ist. Dazu gehören beispielsweise der Cyclopropyl-methyl bis -pentyl-, Cyclobutyl-methyl- bis -butyl-, Cyclopentyl-methyl- bis -propyl- sowie Cyclohexyl-methyl- und -ethyl-Rest. Der Rest "(O)" steht für Sauerstoffatom. Unter "Modulation der Apoptose" wird die Inhibierung oder Induktion der Apoptose verstanden.

Die Xanthinderivate der Formel I werden nach bekannten Verfahren hergestellt (US 3,737,433; US 4,108,995; US 4,833,146).

Eine Verfahrensweise besteht beispielsweise darin, daß man ein 3-Alkylxanthin der Formel II, 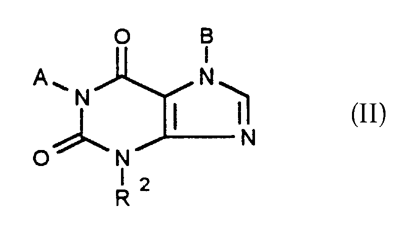 in der
- R²: eine Alkylgruppe mit 1 bis 4 C-Atomen darstellt,
- A: für ein Wasserstoffatom, (C₄ -C₈)-Cycloalkyl-alkyl, (C₂-C₆)-Alkoxyalkyl oder den Rest der Formel II steht und
- B: für ein Wasserstoffatom, (C₄ -C₈)-Cycloalkyl-alkyl, (C₂-C₆)-Alkoxyalkyl, den Rest der Formel II, Benzyl- oder Diphenylrest steht, wobei aber mindestens einer dieser Reste A und B Wasserstoffatom bedeutet,
direkt oder in Gegenwart eines basischen Kondensationsmittels oder in Form eines seiner Salze in 1- oder/und 7-Position einstufig oder stufenweise mit entsprechenden Alkylierungsmitteln der allgemeinen Formel III

X - Q (III)

in der
- X: Halogenatom oder eine Sulfonsäureester- oder Phosphorsäureestergruppierung und
- Q: (C₄ -C₈)-Cycloalkyl-alkyl, (C₂-C₆)-Alkoxyalkyl oder Rest der Formel II bedeutet,
unter nachträglicher reduktiver Abspaltung des Restes B, wenn dieser eine Benzyl- oder Diphenylmethylgruppe darstellt, oder gegebenenfalls hydrolytischer Eliminierung eines Alkoxymethylrestes aus der Position des Restes B und/oder Reduktion der Ketogruppe zur Alkoholfunktion, wenn A oder B einen Oxoalkylrest bedeutet, bei einer Reaktionstemperatur zwischen 0 °C und dem Siedepunkt des jeweils verwendeten Reaktionsmediums alkyliert.

Die Ausgangsstoffe der Umsetzungen sind bekannt oder lassen sich nach literaturbekannten Methoden leicht herstellen.

Bevorzugt ist der Einsatz einer Verbindung der Formel IV und/oder V und/oder eine gegebenenfalls stereoisomeren Form der Verbindung der Formel IV oder V und/oder ein Salz der Verbindung der Formel V, wobei
- R⁵ für: a) Methyl,
b) Cyclopropyl oder
c) (C₃-C₅)-Alkinyl steht,
- R⁶ für: -CF₃ oder -CN steht,
- R⁷: für ein Wasserstoffatom oder Methyl steht, und
- X für: eine -CH- Gruppe steht, in Kombination mit Xanthinderivaten der Formel I, wobei
- R²: für (C₁-C₂)-Alkyl steht,
- R¹: für einen Rest der Formel II steht, worin R für
a) einen Rest -CO- oder
b) einen Rest -C(R⁴)(OH)- steht,
und n die ganze Zahl 3, 4, 5 oder 6 bedeutet und
- R⁴: für ein Wasserstoffatom oder (C₁-C₂)-Alkyl steht und
- R³ für: (C₂-C₅)-Alkyl oder (C₄-C₆)-Cycloalkyl-alkyl steht.

Insbesondere bevorzugt ist die Verwendung von N-(4-Trifluormethylphenyl)-2-cyan-3-hydroxy-crotonsäureamid, 2-Cyano-3-cyclopropyl-3-hydroxy-acrylsäure-(4-cyanophenyl)-amid oder N-(4-Trifluormethylphenyl)-2-cyan-3-hydroxy-hept-2-en-6-in-carbonsäureamid in Kombination mit 1-(5-Hydroxy-5-methylhexyl)-3-methyl-7-propylxanthin.

Die Herstellung der Verbindung der Formel IV oder V erfolgt nach bekannten Verfahren wie sie in EP 484 223; EP 529 500; US 4 061 767; EP 538 783 oder EP 551 230 beschrieben werden. Die Ausgangsstoffe der chemischen Umsetzungen sind bekannt oder lassen sich nach literaturbekannten Methoden leicht herstellen.

Unter dem Begriff Alkyl, Alkenyl oder Alkinyl werden Reste verstanden, deren Kohlenstoffkette geradkettig oder verzweigt sein kann. Ferner können die Alkenyl- oder Alkinyl-Reste auch mehrere Doppelbindungen beziehungsweise mehrere Dreifachbindungen enthalten. Cyclische Alkylreste sind beispielsweise 3- bis 5-gliedrige Monocyclen wie Cyclopropyl, Cyclobutyl oder Cyclopentyl. Unter dem Begriff "überadditiv" werden Wirkungen verstanden, die größer als die Summe der Einzelwirkungen sind.

Das erfindungsgemäße Kombinationspräparat eignet sich beispielsweise zur Behandlung von Transplantationen, Autoimmunerkrankungen, Infarkt, Stroke, Entzündungen, Neurodegeneration, Myome, Muskelatrophie, Muskeldystrophie, Kachexie, Systemic Inflammation Response Syndrome (SIRS), Adult Respiratory Distress Syndrome (ARDS), zerebrale Malaria, chronische Lungenentzündung, Lungensarkosidose, Reperfusionsschäden, Narbenbildung, Darmentzündungen, Verbrennungsschäden, Acquired Immune Deficiency Syndrome (AIDS), Krebs, Erkrankungen mit erhöhten Proteinverlust, chronische Niereninsuffizienz oder hypertrophischen Erkrankungen.

Das erfindungsgemäße Kombinationspräparat kann auch Kompositionen oder Kombinationspackungen umfassen, in denen die Bestandteile nebeneinander gestellt sind und deshalb gleichzeitig, getrennt oder zeitlich abgestuft an ein und denselben menschlichen oder tierischen Körper angewendet werden können.

Die Erfindung betrifft auch Verfahren zur Herstellung eines Kombinationspräparats zur Modulation der Apoptose, die dadurch gekennzeichnet sind, daß man mindestens ein Xanthinderivat der Formel I und eine Verbindung der Formel IV oder V mit einem physiologisch annehmbaren Träger und weiteren geeigneten Wirk-, Zusatz- oder Hilfsstoffen in eine geeignete Darreichungsform bringt.

Das erfindungsgemäße Kombinationspräparat kann als Dosiereinheit in Form von Arzneiformen wie Kapseln (einschließlich Mikrokapseln, die im allgemeinen keine pharmazeutischen Träger enthalten), Tabletten einschließlich Dragees und Pillen, oder Zäpfchen vorliegen, wobei bei Verwendung von Kapseln das Kapselmaterial die Funktion des Trägers wahrnehmen und der Inhalt z. B. als Pulver, Gel, Lösung Emulsion oder Dispersion vorliegen kann. Besonders vorteilhaft und einfach ist es jedoch, orale oder perorale Formulierungen mit den beiden Wirkstoffkomponenten 1) und 2) herzustellen, die die berechneten Mengen der Wirkstoffe zusammen mit jedem gewünschten pharmazeutischen Träger enthalten. Auch eine entsprechende Formulierung (Zäpfchen) für die rektale Therapie kann angewandt werden. Ebenso ist die transdermale Applikation in Form von Salben oder Cremes, parenterale (intraperitoneale, subkutane, intramuskuläre) Injektion oder orale Applikation von Lösungen, die die erfindungsgemäßen Kombinationen enthalten, möglich. Salben, Pasten, Cremes und Puder können neben den Wirkstoffe die üblichen Trägerstoffe enthalten, z. B. tierische und pflanzliche Fette, Wachse, Paraffine, Stärke, Tragant, Cellulosederivate, Polyethylenglykole, Silicone, Kieselsäure, Aluminiumhydroxid, Talkum, Zinkoxid, Milchzucker, Bentite, Calciumsilikat und Polyamidpulver oder Gemische dieser Stoffe. Die Tabletten, Pillen oder Granulatkörper können nach Verfahren wie Preß-, Tauch- oder Wirbelbettverfahren oder Kesseldragierung hergestellt werden und enthalten Trägermittel und andere übliche Hilfsstoffe wie Gelatine, Agarose, Stärke (z. B. Kartoffel-, Mais- oder Weizenstärke), Cellulose wie Ethylcellulose, Siliziumdioxid, Magnesiumcarbonat, verschiedene Zucker wie Milchzucker und/oder Calciumphosphate. Die Dragierlösung besteht gewöhnlich aus Zucker und/oder Stärkesirup und enthält meistens noch Gelatine, synthetische Celluloseester, Gummi arabicum, Polyvinylpyrrolidon, Pigmente, oberflächenaktive Substanzen, Weichmacher und ähnliche Zusätze entsprechend dem Stand der Technik. Zur Herstellung der Zubereitungsformen kann jedes übliche Fließregulierungs-, Schmier- oder Gleitmittel wie Magnesiumstearat und Trennmittel verwendet werden. Bevorzugt haben die Zubereitungen die Form von Mantel-/Kern-Tabletten oder Mehrschichttabletten, wobei sich die Wirkkomponente 2 im Mantel bzw. im Kern bzw. in einer Schicht befindet, während sich die Wirkkomponente 1 im Kern, im Mantel oder in einer anderen Schicht befindet. Die Wirkstoffkomponenten können auch in retardierter Form vorliegen oder an Retardierungsmaterial adsorbiert bzw. im Retardierungsmaterial (z. B. Cellulose- oder Polystyrolharzbasis, z. B. Hydroxyethylcellulose) eingeschlossen sein. Eine verzögerte Freisetzung der Wirkstoffe kann auch erreicht werden, indem die betreffende Schicht bzw. das Kompartiment mit üblichen magensaftunlöslichen Überzügen versehen wird. Die anzuwendende Dosierung ist selbstverständlich abhängig von verschiedenen Faktoren wie dem zu behandelnden Lebewesen (d. h. Mensch oder Tier), Alter, Gewicht, allgemeiner Gesundheitszustand, dem Schweregrad der Symptome, der zu behandelnden Erkrankung, eventuellen Begleiterkrankungen, (falls vorhanden) der Art der begleitenden Behandlung mit anderen Arzneimitteln, oder Häufigkeit der Behandlung. Die Dosierungen werden im allgemeinen mehrfach pro Tag und vorzugsweise einmal bis dreimal pro Tag verabreicht. Die verwendeten Mengen an Einzelwirkstoff orientieren sich hierbei an der empfohlenen Tagesdosis des jeweiligen Einzelwirkstoffs und sollen im allgemeinen im Kombinationspräparat von 10 % bis 100 % der empfohlenen Tagesdosis liegen, bevorzugt von 20 % bis 80 %, insbesondere bei 50 %. Die geeignete Therapie mit den erfindungsgemäßen Kombinationen besteht somit z.B. in der Verabreichung von einer, zwei oder 3 Einzeldosierungen der Zubereitung bestehend aus N-(4-Trifluormethylphenyl-2-cyan-3-hydroxy-crotonsäureamid-Natriumsalz in einer Menge von 2 mg bis 250 mg, bevorzugt 5 mg bis 150 mg, insbesondere 10 mg bis 50 mg, insbesondere bevorzugt 10 mg bis 20 mg und 1-(5-Hydroxy-5-methylhexyl)-3-methyl-7-propylxanthin in einer Menge von 100 bis 600 mg, insbesondere von 150 bis 300 mg, vorzugsweise von 20 bis 200 mg.

### Beispiel 1

### Herstellung von 1-(5-Hydroxy-5-methylhexyl)-3-methyl-7-propylxanthin

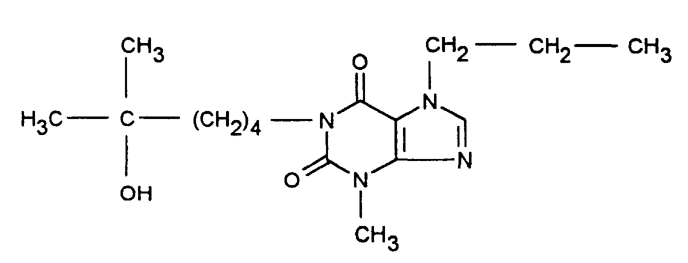

Zu einer Suspension von 61,3 g (0,2 Mol) 3-Methyl-1-(5-oxohexyl)-7-propylxanthin in 2 I wasserfreien Ethers fügt man unter kräftigem Rühren bei Raumtemperatur 22,4 g (0,3 Mol) Methylmagnesiumchlorid in Form einer 20 %igen Lösung in Tetrahydrofuran tropfenweise hinzu, wobei die Innentemperatur bis auf etwa 30 °C ansteigt. Anschließend wird 2 Stunden unter Rühren und Rückfluß erwärmt, zur Zerlegung des gebildeten Alkanolats mit gesättigter wäßriger Ammoniumchlorid-Lösung versetzt, die organische Phase abgetrennt und zweimal mit je 500 ml Wasser gewaschen. Die gesammelten Wasserphasen werden nochmals gründlich mit Dichlormethan extrahiert. Man vereinigt den Dichlormethan-Extrakt mit der etherischen Phase, trocknet über Natriumsulfat, filtriert und dampft unter vermindertem Druck ein, wobei 59,0 g Rohprodukt (91,5 % der Theorie) erhalten werden, die man durch Umkristallisation aus Diisopropylether reinigt. :

| | | |
|---|---|---|
| Ausbeute | 49,8 g (77,2 % der Theorie); C₁₆H₂₆N₄O₃ (MG = 322,4) | Schmelzpunkt: 81 - 82 °C |

| | | | | |
|---|---|---|---|---|
| Analyse | Berechnet | C 59,61 % | H 8,13 % | N 17,38 % |
| | Gefunden | C 59,72 % | H 8,09 % | N 17,44 % |

### Beispiel 2

### Pharmakologische Prüfung

### 2.1 Zellkultur

Die murine Makrophagenzellinie RAW 264.7 wurde von ATCC (Rockville, MD) bezogen und in DMEM (Sigma, St. Louis, MO) mit 4.5 g Glucose/l, 110 mg Natriumpyruvat/l, 10 % Hitze inaktiviertem FCS (Gibco, Grand Island, NY) und Penicillin/Streptomycin (50 U/ 50 mg/ml) kultiviert.

Die Makrophagen wurden alle 2 - 3 Tage passagiert und einen Tag vor Beginn des Experiments zu 2x10⁶ Zellen in Gewebekulturflaschen (75 cm², Falcon, Becton Dickinson GmbH, Heidelberg, Deutschland) ausgebracht. Die Zellen wurden mit frischem Medium versorgt und die Präparate in den entsprechenden Konzentrationen zugesetzt. 1-(5-Hydroxy-5-methylhexyl)-3-methyl-7-propylxanthin (Verbindung 1) wurde 20 mM in Zellmedium gelöst. Davon wurden 100 µl (100 µM) und 50 µl (50 µM) zu 20 ml Medium pipettiert. N-(4-Trifluormethylphenyl-2-cyan-3-hydroxy-crotonsäureamid-Natriumsalz (Verbindung 2) wurde 12 mM in Zellmedium gelöst. Davon wurden je 100 µl (60 µM Endkonzentration), 33 µl (20 µM Endkonzentration) und 16,7 µl (10 µM Endkonzentration) zu 20 ml Medium pipettiert. Die Stimulation mit Lipopolysacchariden (LPS; E. coli, Serotype 0127: B 8 Sigma, St. Louis, MO) in einer Konzentration von 10 ng/ml wurde 1 Stunde nach der Vorinkubation mit dem Präparat durchgeführt. Aliquotes einer Stammlösung von Lipopolysacchariden (LPS 1 mg/ml in 10 % Dimethylsulfoxid (DMSO)) wurden mit Medium auf eine Konzentration von 1 µg/ml verdünnt und bei- 20 °C gelagert. Die Zellen wurden für 24 Stunden (h) bei 37 °C in 10 % CO₂ inkubiert.

### 2.2 Probenvorbereitung

Alle verwendeten Chemikalien waren analytisch rein oder in Elektrophoresequalität und wurden von Millipore Co. (Bedford, MA) oder Sigma (St. Louis, MO) bezogen, wenn nicht gesondert auf andere Bezugsquellen hingewiesen wird.

Die 2-D-Elektrophorese (2-DE) wurde mit dem Investigator System® (Millipore) durchgeführt, und die Proben wurden nach der Vorschrift des Herstellers mit kleinen Veränderungen aufgearbeitet. Die adhärenten murinen Makrophagen wurden auf Eis stehend dreimal je 60 Sekunden mit 10 ml eiskaltem PBS gewaschen. Anschließend wurden die Zellen in 1 ml kochendem Lysispuffer, bestehend aus 0,3 g SDS, 3,088 g DTT, 0,444 g TrisHCl und 0,266 g Tris Base, in 100 ml lysiert. Das Zellysat wurde abgeschabt und in einem 2 ml Probengefäß für 10 Minuten (min) in kochendem Wasser erhitzt.

Polynucleotide wurden durch Zusatz von Benzonase® (Merck, Darmstadt, Deutschland) in 30 min bei 37 °C gespalten.

An dieser Stelle der Probenvorbereitung wurde ein Aliquot entnommen, und der Proteingehalt nach der Methode von Popov bestimmt.

Für die 2-DE wurden die Proteine der Probe durch tropfenweise Zugabe zu eiskaltem Aceton (80 % v/v) ausgefällt. Die Probe wurde für 20 min auf Eis gekühlt und anschließend bei 240 g 10 min zentrifugiert. Das angetrocknete Pellet wurde in einem Teil Lysispuffer und vier Teilen eines Probenpuffers zu einem Proteingehalt von 5 mg/ml aufgenommen. Der Probenpuffer besteht aus 59,7 g Harnstoff, 4,0 ml NP-40, 1,54 g DTT, 5,5 ml Trägerampholyten (pH 3-10, 2-DE optimiert) in 100 ml. Ungelöstes Material wurde vor der Elektrophorese durch Zentrifugation der Proben bei 16000 x g abgetrennt.

### 2.3 2-DE Gel-Elektrophorese

Die hochauflösende Zwei-Dimensionale Gel-Elektrophorese wurde nach der Methode von O'Farrell mit Modifikationen, wie sie von Garrels beschrieben wurden, durchgeführt. Dazu wurde das Millipore Investigator® 2-D Elektrophorese System (Millipore Co., Bedford, MA) eingesetzt.

Die isoelektrische Fokussierung wurde in Glaskapillaren (1 mm im Durchmesser) mit einem 0,08 mm dicken Faden, der ein Dehnen und Reißen des Stäbchens verhindert, durchgeführt.

Das IEF-Gel besteht aus einer 4,1 % T, 2,4 % C Polyacrylamidmatrix, die aus einer 30,8 % T, 2,6 % C Stammlösung hergestellt wurde, 9,5 M Harnstoff, 2,0 % (v/v) NP-40, 10 mM Chaps und 2 % (v/v) Trägerampholyten (pH 3-10, 2-DE optimiert).

Als Anodenpuffer wurde 0,01 M H₃PO₄, als Kathodenpuffer 0,1 M NaOH benutzt. Vor der Vorfokussierung zur Ausbildung des pH-Gradienten wurden 15 µl eines Probenüberschichtungspuffers, bestehend aus 0,5 M Harnstoff, 0,2 % (v/v) NP-40, 0,1 % (v/v) Trägerampholyten und 50 mM DTT, appliziert. Das Spannungsmaximum von 1500 Volt wurde innerhalb von 90 Minuten bei einem maximalen Strom von 110 µA/Gel erreicht. Nach der Vorfokussierung wurden 20 µl der Probe (100 µg Protein) und weitere 15 µl Überschichtungspuffer aufgetragen.

Die isoelektrische Fokussierung der Proteine erfolgte innerhalb von 18000 Vh. Nach Beendigung der Elektrophorese wurden die Stäbchen auf Eis gekühlt und in einem Puffer, bestehend aus 0,3 M Tris Base, 0,075 M Tris HCI, 6 % SDS, 50 mM DTT und 0,01 % Bromphenolblau, äquilibriert. Die Stäbchengele wurden direkt auf die Oberfläche des Vertikalgels der zweiten Dimension überführt oder bis zum Gebrauch bei - 20 °C gelagert. Die zweite Dimension wurde in einem SDS-Gradientengel (10 - 17 %) ohne Sammelgel durchgeführt. Der Gradient wurde durch Mischen zweier Gellösungen hergestellt.
A: 100 ml Acrylamid (30,5 % T, 1,64 % C), 73 ml Tris (1,5 M, pH 8,8), 123 ml H₂O, 3 ml SDS (10 %), 150 µl TEMED und 750 µl Ammoniumperoxodisulfat (10 %).
B: 170 ml Acrylamid, 73 ml Tris, 66,78 g Glycerin, 3 ml SDS, 150 µl TEMED, 750 µl Ammoniumperoxodisulfat.

Die Elektrophorese wurde über Nacht bei konstanter Temperatur in einem Laufpuffer, bestehend aus 25 mM Tris-Base, 192 mM Glyzin und 0,1 % SDS, durchgeführt bis die Bromphenolblaufront etwa 1 cm vom Ende des Gels entfernt war. Nach Beendigung der Elektrophorese wurden die Proteine im Gel nach Heukeshoven und Dernick mit Silberreagenz angefärbt.

Die Analyse der 2-D-Gele und die Herstellung synthetischer Bilder wurde mit dem Biolmage System (Biolmage Systems Co.) durchgeführt. Das erhaltene Proteinmuster wurde von einer Kodak Megaplus Camera Model 1,4 gescannt und die Daten wurden von einer HAM-Station prozessiert.

### 2.4 Ergebnisse

Die Ergebnisse der unstimulierten Kontrolle wurden gleich 100 % gesetzt. Die Zugabe von LPS (10 ng/m)führte zu einer 50 % Dephosphorylierung von Cofilin. Die gleichzeitige Applikation von LPS (10 ng/ml) und Verbindung 1 (100 µM) führt zu einer 10 %igen Dephosphorylierung von Cofilin. Daher ist die Hemmung der Dephosphorylierung 80 % im Vergleich mit den nur mit LPS behandelten Makrophagen.

Tabelle 1 zeigt die Ergebnisse. Im Gegensatz zu 100 µM Endkonzentration sind 50 µM Endkonzentration der Verbindung 1 unwirksam. Die Verbindung 2 ist bis 20 µM Endkonzentration ebenfalls ohne Wirkung, bei 60 µM allein ist sie wirksam. Kombiniert man Verbindung 1 und Verbindung 2 in einem Konzentrationsbereich in dem jede einzelne Verbindung unwirksam ist, ergibt sich überraschender Weise eine überadditive Wirkung.

**Tabelle 1:**

| RAW 264,7 murine Makrophagen | Intensität des Cofilinflecks (%) | Hemmung der Intensitätsabnahme (%) |
|---|---|---|
| Kontrolle (unstimuliert) | 100 | 0 |
| LPS (10 ng/ml) | 50 | 0 |
| LPS + Verb. 1 (50 µM) | 50 | 0 |
| LPS + Verb. 1 (100 µM) | 55 | 10 |
| LPS + Verb. 2 (10 µM) | 50 | 0 |
| LPS + Verb. 2 (20 µM) | 50 | 0 |
| LPS + Verb. 1 (50 µM)+ Verb. 2 (10 µM) | 60 | 20 |
| LPS + Verb. 1 (50 µM)+ Verb. 2 (20 µM) | 90 | 80 |
| Verb. steht für Verbindung | | |

## Patentansprüche

1. Kombinationspräparat, enthaltend
1) ein Xanthinderivat der Formel I 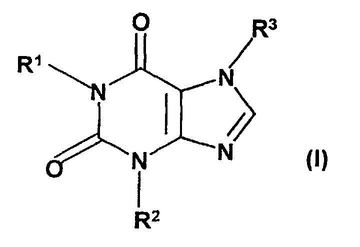 und/oder eine gegebenenfalls stereoisomere Form der Verbindung der Formel I,
wobei R² für -(C₁-C₄)-Alkyl steht,
einer der Reste R¹ oder R³ für einen Rest der Formel II steht,
-(CH₂)ₙ-R-CH₃ (II)
worin
R für
a) eine kovalente Einfachbindung steht und worin n die ganze Zahl Null, 1, 2, 3, 4, 5, 6 oder 7 bedeutet,
b) einen Rest -CO- steht und worin n die ganze Zahl 1, 2, 3, 4, 5 oder 6 bedeutet, oder
c) einen Rest -C(R⁴)(OH)- steht und worin n die ganze Zahl 1, 2, 3, 4, 5 oder 6 bedeutet und
R⁴ für
a) einen Wasserstoffatom oder
b) -(C₁-C₃)-Alkyl steht, und
der andere Rest R³ oder R¹ für
a) ein Wasserstoffatom,
b) (C₁-C₇)-Alkyl,
c) (C₄-C₈)-Cycloalkyl-alkyl oder
d) Alkyl mit 2 bis 6 Kohlenstoffatomen steht, worin die Kohlenstoffkette mit einem Sauerstoffatom unterbrochen ist,
2) eine Verbindung der Formel IV und/oder V, 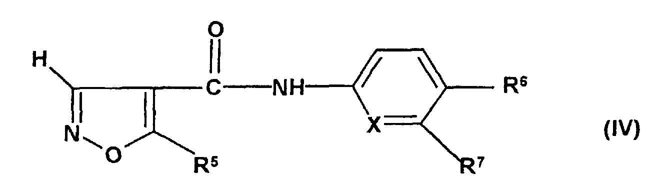 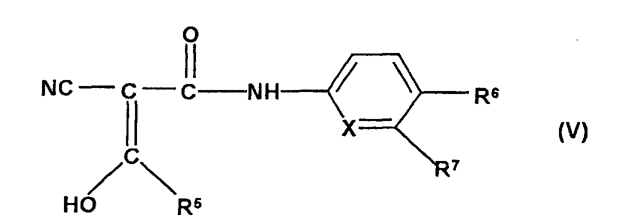 und/oder eine gegebenenfalls stereoisomere Form der Verbindung der Formel IV oder V und/oder ein physiologisch verträgliches Salz der Verbindung der Formel V, wobei
R⁵ für
a) (C₁-C₄)-Alkyl,
b) (C₃-C₅)-Cycloalkyl,
c) (C₂-C₆)-Alkenyl oder
d)(C₂-C₆)-Alkinyl, steht,
R⁶ für
a) -CF₃,
b) -O-CF₃,
c) -S-CF₃,
d) -OH,
e) -NO₂,
f) Halogen,
g) Benzyl,
h) Phenyl,
i) -O-Phenyl,
k) -CN oder
l) -O-Phenyl, ein oder mehrfach substituiert mit
1) (C₁-C₄)-Alkyl,
2) Halogen,
3) -O-CF₃ oder
4) -O-CH₃, steht,
R⁷ für
a) (C₁-C₄)-Alkyl,
b) Halogen, oder
c) ein Wasserstoffatom steht, und
X für
a) eine -CH-Gruppe oder
b) ein Stickstoffatom, steht, und
3) einen pharmazeutischen Träger.

2. Kombinationspräparat gemäß Anspruch 1, **dadurch gekennzeichnet, daß** man eine Verbindung der Formel I einsetzt, wobei
R² für -(C₁-C₄)-Alkyl steht und
einer der Reste R¹ oder R³ für einen Rest der Formel II steht, worin R für
a) einen Rest -CO- oder
b) einen Rest -C(R⁴)(OH)- steht,
und n die ganze Zahl 3, 4, 5 oder 6 bedeutet und
R⁴ für ein Wasserstoffatom oder (C₁-C₃)-Alkyl steht und
der andere Rest R³ oder R¹ für (C₁-C₇)-Alkyl oder (C₄-C₈)-Cycloalkyl-alkyl steht.

3. Kombinationspräparat gemäß Anspruch 1 oder 2, **dadurch gekennzeichnet, daß** man ein Xanthinderivat der Formel I einsetzt, wobei
R² für (C₁-C₂)-Alkyl steht,
R¹ für den Rest der Formel II steht, worin R für
a) einen Rest -CO- oder
b) einen Rest -C(R⁴)(OH)-steht,
und n die ganze Zahl 3, 4, 5 oder 6 bedeutet und R⁴ für ein Wasserstoffatom oder (C₁-C₂)-Alkyl steht und
R³ für (C₁-C₇)-Alkyl oder (C₄-C₈)-Cycloalkyl-alkyl steht.

4. Kombinationspräparat gemäß einem oder mehreren der Ansprüche 1 bis 3, **dadurch gekennzeichnet, daß** man ein Xanthinderivat der Formel I einsetzt,
wobei
R² für (C₁-C₂)-Alkyl steht,
R¹ für einen Rest der Formel II steht, worin R für
a) einen Rest -CO- oder
b) einen Rest -C(R⁴)(OH)- steht,
und n die ganze Zahl 3, 4, 5 oder 6 bedeutet und
R⁴ für ein Wasserstoffatom oder (C₁-C₂)-Alkyl steht und
R³ für (C₂-C₅)-Alkyl oder (C₄-C₆)-Cycloalkyl-alkyl steht.

5. Kombinationspräparat gemäß einem oder mehreren der Ansprüche 1 bis 4, **dadurch gekennzeichnet, daß** man 1-(5-Hydroxy-5-methylhexyl)-3-methyl-7-propylxanthin einsetzt.

6. Kombinationspräparat gemäß Anspruch 1, **dadurch gekennzeichnet, daß** man eine Verbindung der Formel IV und/oder V, wobei
R⁵ für
a) Methyl,
b) Cyclopropyl oder
c) (C₃-C₅)-Alkinyl steht,
R⁶ für -CF₃ oder -CN steht,
R⁷ für Wasserstoffatom oder Methyl steht, und
X für eine -CH- Gruppe steht in Kombination mit Xanthinderivaten der Formel I einsetzt, wobei
R² für (C₁-C₂)-Alkyl steht,
R¹ für einen Rest der Formel II steht, worin R für
a) einen Rest -CO- oder
b) einen Rest -C(R⁴)(OH)-steht,
und n die ganze Zahl 3, 4, 5 oder 6 bedeutet, und
R⁴ für ein Wasserstoffatom oder (C₁-C₂)-Alkyl steht und
R³ für (C₂-C₅)-Alkyl oder (C₄-C₆)-Cycloalkyl-alkyl steht.

7. Kombinationspräparat gemäß Anspruch 1, **dadurch gekennzeichnet, daß** man als Verbindung der Formel V N-(4-Trifluormehtylphenyl)-2-cyan-3-hydroxycrotonsäureamid, 2-Cyano-3-cyclopropyl-3-hydroxy-acrylsäure-(4-cyanophenyl)-amid oder N-(4-Trifluormethylphenyl)-2-cyan-3-hydroxy-hept-2-en-6-in-carbonsäureamid und als Xanthinderivat 1-(5-Hydroxy-5-methylhexyl)-3-methyl-7-propylxanthin einsetzt.

8. Kombinationspräparat gemäß Anspruch 1, **dadurch gekennzeichnet, daß** man N-(4-Trifluormethylphenyl)-2-cyan-3-hydroxy-crontonsäureamid und 1-(5-Hydroxy-5-methylhexyl)-3-methyl-7-propylxanthin einsetzt.

9. Verwendung des Kombinationspräparats gemäß einem oder mehreren der Ansprüche 1 bis 8 zur Herstellung eines Arzneimittels zur Modulation der Apoptose.

10. Verwendung gemäß Anspruch 9 zur Herstellung eines Arzneimittels zur Behandlung von Transplantationen, Autoimmunerkrankungen, Infarkt, Stroke, Entzündungen, Neurodegeneration, Myome, Muskelatrophie, Muskeldystrophie, Kachexie, Systemic Inflammation, Response Syndrome (SIRS), Adult Respiratory Distress Syndrome (ARDS), zerebrale Malaria, chronische Lungenentzündung, Lungensarkosidose, Reperfusionsschäden, Narbenbildung, Darmentzündungen, Verbrennungsschäden, Acquired Immune Deficiency Syndrome (AIDS), Krebs, Erkrankungen mit erhöhtem Proteinverlust, chronische Nierensuffizienz oder hypertrophischen Erkrankungen.

## Claims

1. A combination preparation comprising
1) a xanthine derivative of the formula I 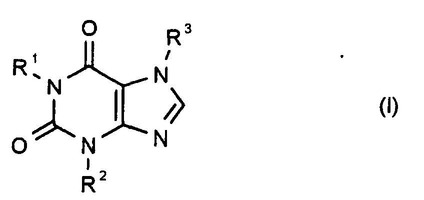 and/or an optionally stereoisomeric form of the compound of the formula I,
where R² is -(C₁-C₄)-alkyl,
one of the radicals R¹ or R³ is a radical of the formula II
-(CH₂)ₙ-R-CH₃ (II)
in which
R
a) is a covalent single bond and in which n is the integer zero, 1, 2, 3, 4, 5, 6 or 7,
b) is a radical -CO- and in which n is the integer 1, 2, 3, 4, 5 or 6, or
c) is a radical -C(R⁴)(OH)- and in which n is the integer 1, 2, 3, 4, 5 or 6 and
R⁴ is
a) a hydrogen atom or
b) -(C₁-C₃)-alkyl, and
the other radical R³ or R¹ is
a) a hydrogen atom,
b) (C₁-C₇)-alkyl,
c) (C₄-C₈)-cycloalkylalkyl or
d) alkyl having 2 to 6 carbon atoms, in which the carbon chain is interrupted by an oxygen atom,
2) a compound of the formula IV and/or V, 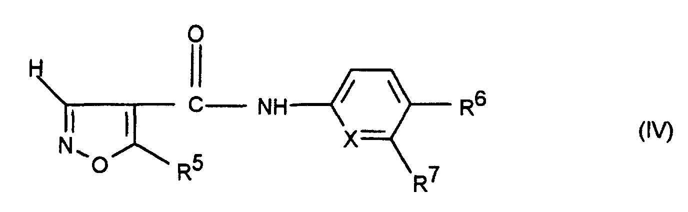 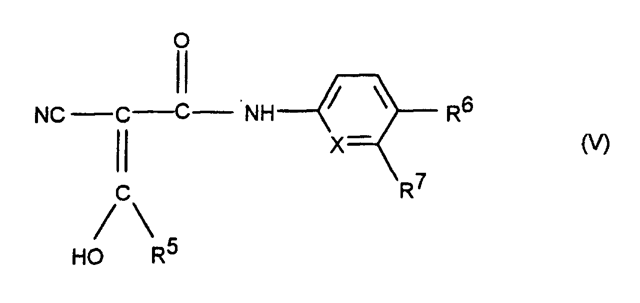 and/or an optionally stereoisomeric form of the compound of the formula IV or V and/or a physiologically tolerable salt of the compound of the formula V, where
R⁵ is
a) (C₁-C₄)-alkyl,
b) (C₃-C₅)-cycloalkyl,
c) (C₂-C₆)-alkenyl or
d) (C₂-C₆)-alkynyl,
R⁶ is
a) -CF₃,
b) -O-CF₃,
c) -S-CF₃,
d) -OH,
e) -NO₂,
f) halogen,
g) benzyl,
h) phenyl,
i) -O-phenyl,
k) -CN or
l) -O-phenyl, mono- or polysubstituted by
1) (C₁-C₄)-alkyl,
2) halogen,
3) -O-CF₃ or
4) -O-CH₃,
R⁷ is
a) (C₁-C₄)-alkyl,
b) halogen, or
c) a hydrogen atom, and
X is
a) a -CH group or
b) a nitrogen atom, and
3) a pharmaceutical excipient.

2. A combination preparation as claimed in claim 1, wherein a compound of the formula I is employed where
R² is -(C₁-C₄)-alkyl and
one of the radicals R¹ or R³ is a radical of the formula II, in which R is
a) a radical -CO- or
b) a radical -C(R⁴)(OH)-,
and n is the integer 3, 4, 5 or 6 and
R⁴ is a hydrogen atom or (C₁-C₃)-alkyl and
the other radical R³ or R¹ is (C₁-C₇)-alkyl or (C₄-C₈)-cycloalkylalkyl.

3. A combination preparation as claimed in claim 1 or 2, wherein a xanthine derivative of the formula I is employed where
R² is (C₁-C₂)-alkyl,
R¹ is the radical of the formula II, in which R is
a) a radical -CO- or
b) a radical -C(R⁴)(OH)-,
and n is the integer 3, 4, 5 or 6 and
R⁴ is a hydrogen atom or (C₁-C₂)-alkyl and
R³ is (C₁-C₇)-alkyl or (C₄-C₈)-cycloalkylalkyl.

4. A combination preparation as claimed in one or more of claims 1 to 3, wherein a xanthine derivative of the formula I is employed where
R² is (C₁-C₂)-alkyl,
R¹ is a radical of the formula II in which R is
a) a radical -CO- or
b) a radical -C(R⁴)(OH)-,
and n is the integer 3, 4, 5 or 6 and
R⁴ is a hydrogen atom or (C₁-C₂)-alkyl and
R³ is (C₂-C₅)-alkyl or (C₄-C₆)-cycloalkylalkyl.

5. A combination preparation as claimed in one or more of claims 1 to 4, wherein 1-(5-hydroxy-5-methylhexyl)-3-methyl-7-propylxanthine is employed.

6. A combination preparation as claimed in claim 1, wherein a compound of the formula IV and/or V is employed where
R⁵ is
a) methyl,
b) cyclopropyl or
c) (C₃-C₅)-alkynyl,
R⁶ is -CF₃ or -CN,
R⁷ is a hydrogen atom or methyl, and
X is a -CH- group, in combination with xanthine derivatives of the formula I, where
R² is (C₁-C₂)-alkyl,
R¹ is a radical of the formula II, in which
R is
a) a radical -CO-, or
b) a radical -C(R⁴)(OH)-,
and n is the integer 3, 4, 5 or 6 and
R⁴ is a hydrogen atom or (C₁-C₂)-alkyl and
R³ is (C₂-C₅)-alkyl or (C₄-C₆)-cycloalkylalkyl.

7. A combination preparation as claimed in claim 1, wherein the compound of the formula V employed is N-(4-trifluoromethylphenyl)-2-cyano-3-hydroxycrotonamide, 2-cyano-3-cyclopropyl-3-hydroxyacrylic acid (4-cyanophenyl)amide or N-(4-trifluoromethylphenyl)-2-cyano-3-hydroxyhept-2-en-6-yne-carboxamide and the xanthine derivative employed is 1-(5-hydroxy-5-methylhexyl)-3-methyl-7-propylxanthine.

8. A combination preparation as claimed in claim 1, wherein N-(4-trifluoromethylphenyl)-2-cyano-3-hydroxycrotonamide and 1-(5-hydroxy-5-methylhexyl)-3-methyl-7-propylxanthine are employed.

9. The use of the combination preparation as claimed in one or more of claims 1 to 8 for the production of a pharmaceutical for the modulation of apoptosis.

10. The use as claimed in claim 9 for the production of a pharmaceutical for the treatment of transplants, autoimmune disorders, infarct, stroke, inflammations, neurodegeneration, myoma, muscular atrophy, muscular dystrophy, cachexia, systemic inflammation response syndrome (SIRS), adult respiratory distress syndrome (ARDS), cerebral malaria, chronic pneumonia, pulmonary sarcosidosis, reperfusion damage, scar formation, enteritis, burn damage, acquired immune deficiency syndrome (AIDS), cancer, disorders with increased protein loss, chronic renal insufficiency or hypertrophic disorders.

## Revendications

1. Préparation d'une combinaison, contenant
1) un dérivé de xanthine de formule I 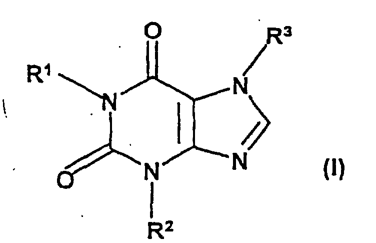 et/ou une forme éventuellement stéréoisomère du composé de formule I,
dans laquelle R² représente un groupe alkyle en C₁ à C₄, un des groupes R¹ ou R³ représente un groupe de formule II,
- (CH₂)ₙ-R-CH₃ (II)
dans laquelle
R représente
a) une simple liaison covalente et dans laquelle n représente le nombre entier zéro, 1, 2, 3, 4, 5, 6 ou 7,
b) un groupe -CO- et dans laquelle n représente le nombre entier 1, 2, 3, 4, 5 ou 6, ou
c) un groupe -C(R⁴)(OH)- et dans laquelle n représente le nombre entier 1, 2, 3, 4, 5 ou 6 et
R⁴ représente
a) un atome d'hydrogène ou
b) un groupe alkyle en C₁ à C₄, et
l'autre groupe R³ ou R¹ représente
a) un atome d'hydrogène,
b) un groupe alkyle en C₁ à C₇,
c) un groupe cycloalkyle en C₄ à C₈-alkyle ou
d) un groupe alkyle ayant de 2 à 6 atomes de carbone, dans lequel la chaîne hydrocarbonée est interrompue par un atome d'oxygène,
2) un composé de formule IV et/ou V, 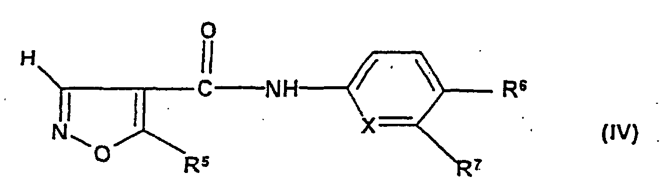 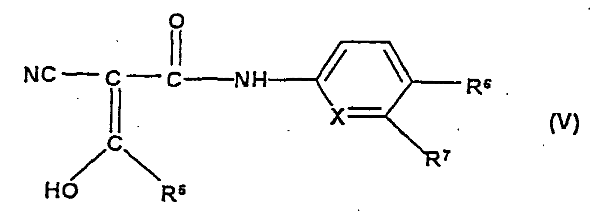 et/ou une forme éventuellement stéréoisomère du composé de formule IV ou V et/ou un sel physiologiquement acceptable du composé de formule V, dans laquelle
R⁵ représente
a) un groupe alkyle en C₁ à C₄,
b) cycloalkyle en C₃ à C₅,
c) alcényle en C₂ à C₆ ou
d) alcynyle en C₂ à C₆,
R⁶ représente
a) -CF₃,
b) -O-CF₃,
c) -S-CF₃,
d) -OH,
e) -NO₂,
f) un halogène,
g) benzyle,
h) phényle,
i) -O-phényle,
k) -CN ou
l) -O-phényle, une ou plusieurs fois substitué avec
1) un alkyle en C₁ à C₄,
2) un halogène
3) -O-CF₃ ou
4) -O-CH₃,
R⁷ représente
a) un alkyle en C₁ à C₄,
b) un halogène, ou
3) un atome d'hydrogène, et
X représente
a) un groupe CH ou
b) un atome d'azote, et
3) un support pharmaceutique.

2. Préparation d'une combinaison selon la revendication 1, **caractérisée en ce qu'**on utilise un composé de formule I, dans laquelle
R² représente un groupe alkyle en C₁ à C₄ et un des groupes R¹ ou R³ représente un groupe de formule II, dans laquelle R représente
a) un groupe -CO- ou
b) un groupe -C(R⁴)(OH)-,
et n le nombre entier 3, 4, 5 ou 6 et
R⁴ représente un atome d'hydrogène ou un groupe alkyle en C₁ à C₃ et
l'autre groupe R³ ou R¹ représente un groupe alkyle en C₁ à C₇ ou cycloalkyle en C₄ à C₈-alkyle.

3. Préparation de combinaison selon la revendication 1 ou 2, **caractérisé en ce qu'**on utilise un dérivé de xanthine de formule I, dans laquelle
R² représente un groupe alkyle en C₁ à C₂,
R¹ représente le groupe de formule II, dans laquelle R représente
a) un groupe -CO- ou
b) un groupe -C(R⁴)(OH)-,
et n le nombre entier 3, 4, 5 ou 6 et
R⁴ représente un atome d'hydrogène ou un groupe alkyle en C₁ à C₂ et
R³ représente un groupe alkyle en C₁ à C₇ ou cycloalkyle en C₄ à C₈-alkyle.

4. Préparation de combinaison selon une ou plusieurs des revendications 1 à 3, **caractérisée en ce qu'**on utilise un dérivé de xanthine de formule I, dans laquelle R² représente un groupe alkyle en C₁ à C₂,
R¹ représente un groupe de formule II, dans laquelle R représente
a) un groupe -CO- ou
b) un groupe -C(R⁴)(OH)-,
et n le nombre entier 3, 4, 5 ou 6 et
R⁴ représente un atome d'hydrogène ou un groupe alkyle en C₁ à C₂ et
R³ représente un groupe alkyle en C₂ à C₅ ou cycloalkyle en C₄ à C₆-alkyle.

5. Préparation de combinaison selon une ou plusieurs des revendications 1 à 4, **caractérisée en ce qu'**on utilise la 1-(5-hydroxy-5-méthylhexyl)-3-méthyl-7-propyl-xanthine.

6. Préparation de combinaison selon la revendication 1, **caractérisée en ce qu'**on utilise un dérivé de xanthine de formule IV et/ou V, dans laquelle
R⁵ représente le groupe
a) méthyle,
b) cyclopropyle ou
c) alcynyle en C₃ à C₅,
R⁶ représente -CF₃ ou -CN,
R⁷ représente un atome d'hydrogène ou un groupe méthyle, et
X représente un groupe -CH- utilisé en combinaison avec des dérivés xanthine de formule I, dans laquelle
R² représente un groupe alkyle en C₁ à C₂,
R¹ représente un groupe de formule II, dans laquelle R représente
a) un groupe -CO- ou
b) un groupe -C(R⁴)(OH)-,
et n le nombre entier 3, 4, 5 ou 6 et
R⁴ représente un atome d'hydrogène ou un groupe alkyle en C₁ à C₂ et
R³ représente un groupe alkyle en C₂ à C₅ ou cycloalkyle en C₄ à C₆-alkyle.

7. Préparation de combinaison selon la revendication 1, **caractérisée en ce qu'**on utilise comme composé de formule V le N-(4-trifluorométhylphényl)-2-cyano-3-hydroxycrotonamide, le 2-cyano-3-cyclopropyl-3-hydroxyacryloyl-(4-cyanophényl)-amide ou le N-(4-trifluorométhylphényl)-2-cyano-3-hydroxy-hept-2-èn-6-yn-carboxamide et comme dérivé xanthine la 1-(5-hydroxy-5-méthylhexyl)-3-méthyl-7-propylxanthine.

8. Préparation de combinaison selon la revendication 1, **caractérisée en ce qu'**on utilise le N-(4-trifluorométhylphényl)-2-cyano-3-hydroxycrotonamide et la 1-(5-hydroxy-5-méthylhexyl)-3-méthyl-7-propylxanthine.

9. Utilisation de la préparation de combinaison selon une ou plusieurs des revendications 1 à 8 pour la préparation d'un médicament pour la modulation de l'apoptose.

10. Utilisation selon la revendication 9 pour la préparation d'un médicament pour le traitement de transplantations, de maladies auto-immunes, d'infarctus, de chocs, d'inflammations, de neurodégénérescence, de myome, d'atrophie musculaire, de dystrophie musculaire, de cachexie, d'inflammation systémique, de syndrome de réponse (SIRS), de syndrome de détresse respiratoire adulte (ARDS), de malaria cérébrale, d'inflammation pulmonaire chronique, de sarcosidose pulmonaire, de troubles de reperfusion, de formation de tubercules, d'inflammations intestinales, de troubles inflammatoires, de Syndrome d'ImmunoDéficience Acquise (SIDA), de cancer, de maladies avec une augmentation de la perte en protéines, d'insuffisance rénale chronique ou de maladies hypertrophiques.
